# EUROPEAN PATENT APPLICATION

(11) **EP 2 957 221 A1**
(43) Date of publication of application: **23.12.2015**
(21) Application number: 15170370.9
(22) Date of filing: 03.06.2015
(51) Int. Cl.: A61B 5/00, A61B 8/08, A61B 8/00

(54) **OBJECT INFORMATION ACQUIRING APPARATUS**

(30) Priority: 20.06.2014 JP 2014127662
(71) Applicant: Canon Kabushiki Kaisha, Tokyo 146-8501 (JP)
(72) Inventor: SATO, Akira, Tokyo, Tokyo 146-8501 (JP); WANDA, Koichiro, Tokyo, Tokyo 146-8501 (JP)
(74) Representative: TBK

(57) **Abstract**

Provided is an object information acquiring apparatus including: detecting elements converting an acoustic wave propagated from an object to an electric signal, a processing unit acquiring characteristic information of the object using the electric signal, a supporting unit supporting the detecting elements, an input unit receiving information concerning three-dimensional region of interest, a display controlling unit displaying a predetermined three-dimensional shape representing, a scanning region setting unit setting a scanning region, and a scanning unit moving the supporting unit, wherein the display controlling unit changes the size of the region of interest stepwise by using a feature value peculiar to the apparatus as a unit based on the inputted information, and displays the changed region of interest.

## Description

### BACKGROUND OF THE INVENTION

### Field of the Invention

The present invention relates to an object information acquiring apparatus.

### Description of the Related Art

As a technique for imaging the inside of an object using an acoustic wave (typically, an ultrasound wave), a photoacoustic imaging method has been proposed. The photoacoustic imaging method is a method of visualizing information related to an optical characteristic value on the inside of an object using an acoustic wave generated by irradiating the object with pulsed laser light.

US Patent No. 5713356 describes a method of receiving an ultrasound wave from an object (a breast) using a plurality of transducers disposed on a hemisphere and generating three-dimensional image data (image reconstruction). In an apparatus described in US Patent No. 5713356, an examinee inserts a breast into a detector, in which hemispherical transducers are disposed, and takes a face-down posture. Water for taking acoustic matching is filled between the inserted breast and the transducers. During measurement, the detector, in which the transducers are provided, rotates stepwise. The transducers receive acoustic waves in positions in respective steps. By scanning the detector in this way, even with a small number of the transducers, it is possible to perform the measurement as if the transducers were present in many directions. The breast is inserted to be located near the hemispherical center of the detector. Pulsed light is irradiated from a hemispherical vertex portion of the detector.

Patent Literature 1: US Patent No. 5713356

### SUMMARY OF THE INVENTION

In the case of the apparatus that scans the detector as in US Patent No. 5713356, it is desired that a user can designate a region where the user desires to acquire characteristic information in the object (a region of interest (ROI)) out of an entire region that can be scanned. In particular, in the case of an apparatus that takes an excessively long time to measure the entire region, in order to reduce the measurement time, there is a demand for a function for performing measurement of only a designated region of interest.

In an apparatus that generates three-dimensional image data like the apparatus of US Patent No. 5713356, it is preferable that the region of interest can also be three-dimensionally designated. However, since conventional setting operation for a three-dimensional region of interest is complicated, there is a demand for a method of simply designating a three-dimensional region.

The present invention has been devised in view of such problem recognition. The present invention was developed to provide a technique for enabling a user to easily set a three-dimensional region of interest.

The present invention in its first aspect provides an object information acquiring apparatus as specified in claims 1 to 11.

According to the present invention, it is possible to provide a technique for enabling a user to easily set a three-dimensional region of interest.

Further features of the present invention will become apparent from the following description of exemplary embodiments with reference to the attached drawings.

### BRIEF DESCRIPTION OF THE DRAWINGS

FIG. 1 is a block diagram showing the configuration of an apparatus according to a first embodiment;
FIGS. 2A and 2B are diagrams showing a sensitivity characteristic of an acoustic detecting element according to the first embodiment;
FIG. 3 is an example of an initial screen for designating a region of interest according to the first embodiment;
FIG. 4 is an XY sectional view of a typical shape of the region of interest according to the first embodiment;
FIG. 5 is an explanatory diagram of an XY sectional shape of the typical shape of the region of interest according to the first embodiment;
FIG. 6 is a size change explanatory diagram of the typical shape of the region of interest according to the first embodiment; and
FIG. 7 is a flowchart for explaining processing according to the first embodiment.

### DESCRIPTION OF THE EMBODIMENTS

A preferred embodiment of the present invention is explained below with reference to the drawings. However, the dimensions, the materials, the shapes, a relative arrangement, and the like of components described below should be changed as appropriate according to the configuration and various conditions of an apparatus applied with the invention and are not meant to limit the scope of the present invention to the description explained below.

The present invention relates to a technique for detecting an acoustic wave propagated from an object, generating characteristic information of the inside of the object, and acquiring the characteristic information. Therefore, the present invention is grasped as an object information acquiring apparatus or a control method therefor, an object information acquiring method, or a signal processing method. The present invention is also grasped as a computer program for causing an information processing apparatus including hardware resources such as a CPU to execute these methods or a storage medium having the computer program stored therein. The present invention is also grasped as an acoustic wave measuring apparatus or a control method therefor.

The object information acquiring apparatus of the present invention includes an apparatus that makes use of a photoacoustic tomography technique for irradiating an object with light (an electromagnetic wave) and receiving (detecting) an acoustic wave generated in a specific position in the object or the surface of the object and propagated according to a photoacoustic effect. Such an object information acquiring apparatus obtains characteristic information inside the object in a form of image data or the like on the basis of photoacoustic measurement. Therefore, the object information acquiring apparatus can be called photoacoustic imaging apparatus and photoacoustic image-forming apparatus.

Characteristic information in a photoacoustic apparatus indicates a generation source distribution of an acoustic wave generated by light irradiation, an initial sound pressure distribution in the object, an optical energy absorption density distribution or an absorption coefficient distribution derived from the initial sound pressure distribution, or a concentration distribution of a substance forming a tissue. Specifically, the characteristic information is an oxygenated/reduced hemoglobin concentration distribution, a blood component distribution such as an oxygen saturation degree distribution calculated from the oxygenated/reduced hemoglobin concentration distribution, a distribution of fat, collagen, or moisture, or the like. The characteristic information may be calculated as distribution information of positions in the object rather than as numerical value data. That is, distribution information of the absorption coefficient distribution, the oxygen saturation degree distribution, or the like may be set as object information.

The acoustic wave in the present invention typically means an ultrasound wave and includes an elastic wave called sound wave or acoustic wave. The acoustic wave generated by the photoacoustic effect is called photoacoustic wave or light-induced ultrasound wave. An electric signal converted from the acoustic wave by a probe is also called acoustic signal.

As the object in the present invention, a breast of a living organism is mainly assumed. However, the object is not limited to this. Measurement of other segments of the living organism and a non-living organism material is also possible.

### [First Embodiment]

### (Spiral scan type)

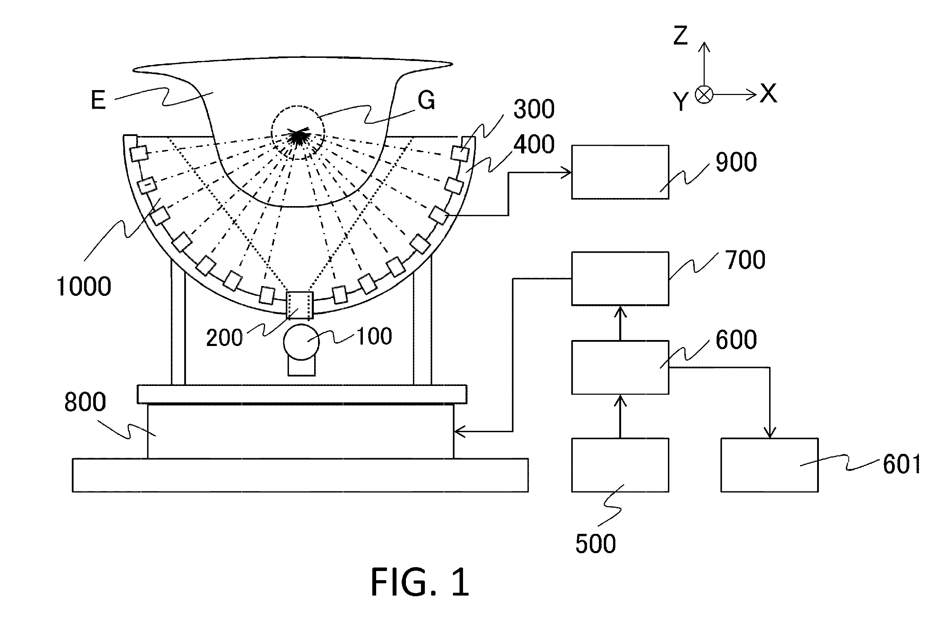

A schematic diagram showing the configuration of a photoacoustic apparatus in a preferred embodiment of the present invention is shown in FIG. 1.

The photoacoustic apparatus (hereinafter also simply referred to as "apparatus") shown in FIG. 1 acquires characteristic information of an object E (a breast) and creates an image of the inside of the object E. The photoacoustic apparatus in this embodiment includes, as components, a light source 100, an optical system 200 functioning as a light irradiating unit, an acoustic detecting element 300, a supporting unit 400, and a region-of-interest designating unit 500 functioning as an input unit. The photoacoustic apparatus further includes a display controlling unit 600, a scanning region setting unit 700, a scanner 800 functioning as a scanning unit, a signal processing unit 900, and an acoustic matching material 1000. Reference numeral 601 denotes a display unit.

The object and the components are explained below.

### (Object)

Although the object E is a target of measurement and is not a component configuring the apparatus, the object E is explained below. Specific examples of the object E include a living organism such as a breast and a phantom simulating an acoustic characteristic and an optical characteristic of the living organism used for adjustment and the like of the apparatus. The acoustic characteristic is typically propagation speed and an attenuation ratio of an acoustic wave. The optical characteristic is typically an absorption coefficient and a scattering coefficient of light. A light-absorbing body having a large light absorption coefficient is present on the inside of the object. In the living organism, hemoglobin, water, melanin, collagen, lipid, and the like are light-absorbing bodies. In the phantom, a substance simulating the optical characteristic is encapsulated on the inside as a light-absorbing body.

### (Light source)

The light source 100 is a device that generates pulsed light. In order to obtain a large output, a laser is desirable as the light source. However, the light source may be a light-emitting diode or the like. In order to effectively generate a photoacoustic wave, it is preferable to irradiate an object with light in a sufficiently short time according to a thermal characteristic of the object. When the object is a living organism, it is desirable to set pulse width of the pulsed light generated from the light source 100 is set to several tens nanoseconds or less. Wavelength of the pulsed light is a near infrared region called window of the living organism and is desirably approximately 700 nm to 1200 nm. Light in this region reaches a relatively deep part of the living organism. Therefore, information concerning a deep part inside the object can be acquired. If the measurement is limited to measurement of a living organism surface portion, visible light of approximately 500 nm to 700 nm to the near infrared region may be used. Further, the wavelength of the pulsed light desirably has a high absorption coefficient with respect to an observation target.

### (Optical system)

The optical system 200 is a device that leads the pulsed light generated by the light source 100 to the object E. Specifically, the optical system 200 is an optical device such as a lens, a mirror, a prism, an optical fiber, or a diffusion plate or a combination of these devices. When the light is led, the shape and the light density of the light are sometimes changed to obtain a desired light distribution using these optical devices. Optical devices are not limited to the optical devices described above and may be any optical devices as long as the optical devices satisfy such functions. The optical system is equivalent to an irradiating unit of the present invention.

Note that, as the intensity of light permitted to be irradiated on a living organism tissue, a maximum permissible exposure (MPE) is decided by a safety standard. As the safety standard, there is IEC 60825-1: Safety of laser products. Besides, there are JIS C 6802: safety standard of laser products, FDA: 21 CFR Part 1040.10, ANSI Z136. 1: Laser Safety Standards, and the like. The maximum permissible exposure specifies the intensity of light that can be irradiated per unit area. Therefore, in order to obtain a satisfactory object internal image while observing the maximum permissible exposure, it is preferable to collectively irradiate light on the surface of the object E in a wide area. Consequently, it is possible to lead a lot of light to the object E while suppressing light intensity per unit area. Therefore, reception of a photoacoustic wave at a high SN ratio is possible. Therefore, it is preferable to spread light to a certain degree of an area as indicated by a broken line extending from the optical system 200 shown in FIG. 1 rather than condensing the light with a lens.

### (Acoustic detecting element)

The acoustic detecting element 300 receives a photoacoustic wave and converts the photoacoustic wave into an electric signal. The acoustic detecting element 300 is desirably an acoustic detecting element having high reception sensitivity and a wide frequency band with respect to the photoacoustic wave from the object E. As a member forming the acoustic detecting element 300, a piezoelectric ceramic material represented by lead zirconate titanate (PZT), a polymer piezoelectric film material represented by polyvinylidene fluoride (PVDF), and the like can be used. An element other than the piezoelectric element may be used. For example, an element of a capacitance type such as a capacitive micro-machined ultrasonic transducer (cMUT), an acoustic detecting element including a Fabry-Perot interferometer, and the like can be used.

The acoustic detecting element 300 has an axial direction in which reception sensitivity increases. A reception region where the photoacoustic wave is received at high sensitivity is formed in the axial direction. In the following explanation, "the axial direction in which reception sensitivity increases" is described as "directional axis".

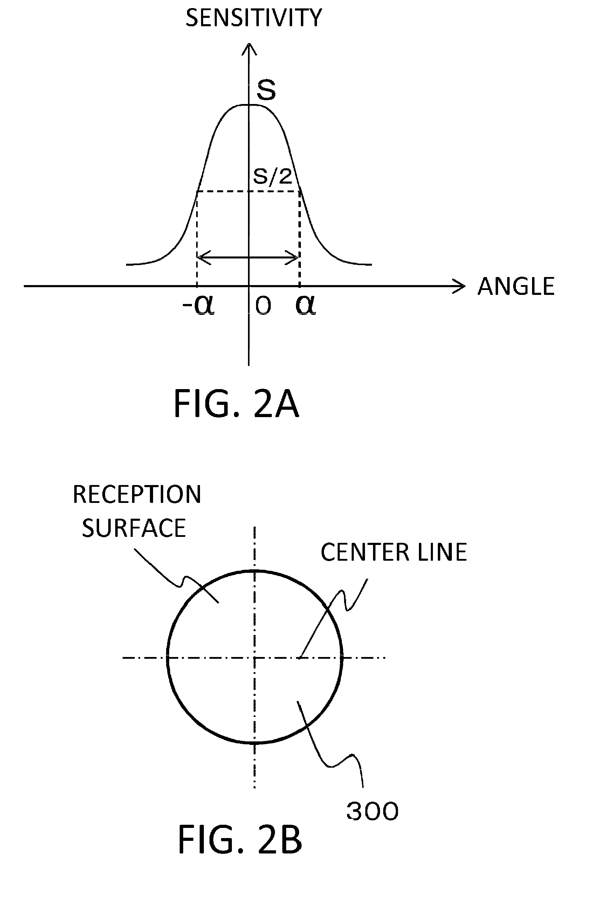

FIG. 2A is a diagram showing an example of a sensitivity characteristic of the acoustic detecting element 300. As shown in FIG. 2B, the acoustic detecting element 300 typically includes a circular planar reception surface. FIG. 2A shows, with an incident angle of incidence of a photoacoustic wave from the normal direction of the reception surface set to 0 degree, a sensitivity characteristic corresponding to the incident angle on a cross section passing the center line of the acoustic detecting element 300 shown in FIG. 2B. In the example shown in FIG. 2A, sensitivity in the case of the incidence from the normal direction of the reception surface is the highest. The sensitivity decreases as the incident angle increases.

The incident angle at the time when the sensitivity is a half S/2 of a maximum S is represented as α. In this embodiment, a region where the photoacoustic wave is made incident on the reception surface of the acoustic detecting element 300 at an angle equal to or smaller than the incident angle α is a region where the photoacoustic wave can be received at high sensitivity. In the following explanation, "the region where the photoacoustic wave can be received at high sensitivity" is described as "high-sensitivity reception region". In this way, the acoustic detecting element 300 has the directional axis. The high-sensitivity reception region where the photoacoustic wave is received at high sensitivity is formed around the directional axis.

Note that the shape of the reception surface of the acoustic detecting element 300 is not limited to this. The reception sensitivity may have a direction characteristic.

### (Supporting unit)

The supporting unit 400 shown in FIG. 1 is a substantially hemispherical container. A plurality of acoustic detecting elements 300 are set on the surface on the inner side of the hemisphere. The optical system 200 is set in a lower part (a pole) of the hemisphere. On the inner side of the hemisphere, a solution is filled as the acoustic matching material 1000 explained below. The acoustic detecting elements 300 are connected to a signal processing unit 900 explained below by a not-shown lead wire. The shape of the supporting unit 400 is not limited to the hemisphere. A spherical crown shape, a shape obtained by cutting a part of an ellipsoid, a shape obtained by combining a plurality of planes or curved surfaces, and the like can also be used. As explained below, the shape of the supporting unit 400 only has to be a shape that can support a plurality of acoustic detecting elements such that directional axes of at least a part of the plurality of acoustic detecting elements converge.

The supporting unit 400 is preferably configured using a metal material having high mechanical strength in order to support these members. It is preferable to provide a sealing member for preventing the acoustic matching material 1000 from leaking to the outer side of the supporting unit 400. A member that holds the object E may be provided in an upper part of the supporting unit 400. The holding member suitably has a cup shape or a bowl shape. The holding member desirably has transmissivity to light from the irradiating unit and a photoacoustic wave from the inside of the object.

When elements are arrayed in an array shape on the hemispherical surface of the supporting unit 400, directional axes of at least a part of the plurality of acoustic detecting elements 300 form an angle different from directional axes of the other elements. The directional axes of the elements cross in a region substantially in the center of the hemisphere. FIG. 1 is a sectional view taken along a vertical surface passing the center of the hemisphere. Alternate long and short dash lines converging on a part of a region in the object E indicate the directional axes of the acoustic detecting elements 300. The optical system 200 is arranged to illuminate the region substantially in the center of the hemisphere. In the case of such an arrangement, in an image obtained by universal back projection, the resolution is high in the center of the hemisphere and decreases away from the center. Position information of the acoustic detecting elements 300 and the optical system 200 in the supporting unit 400 is stored in a not-shown recording device such as a memory and used when an image is generated.

In this specification, such a region having high resolution is referred to as high-resolution region. In this embodiment, the high-resolution region indicates a region from a point where the resolution is the highest to a point where the resolution is a half of the highest resolution. In FIG. 1, a region G is equivalent to the high-resolution region. Note that directions in which sensitivities of the acoustic detecting elements are the highest do not always have to cross as long as directions in which reception sensitivities are high are directed to a specific region and a desired high-resolution region can be formed. A part of the plurality of acoustic detecting elements supported by the supporting unit only have to be directed to the specific region.

### (Region-of-interest designating unit)

The region-of-interest designating unit 500 is input means for the user to designate a three-dimensional region of interest. That is, the region-of-interest designating unit 500 can receive an input of information concerning a region of interest, which is a three-dimensional region. The user designates the region of interest while referring to a photographed image of the object E displayed on the display unit 601. As the photographed image of the object E at this point, an image photographed by a not-shown capture camera is displayed. The region-of-interest designating unit 500 may be a pointing device such as a mouse or a keyboard, a pen tablet type device, or a touch pad attached to the surface of the display unit 601. The display unit 601 may be configured by a touch panel. The region-of-interest designating unit 500 is equivalent to an input unit of the present invention.

Further, on the display unit 601, a typical shape of the region of interest displayed by the display controlling unit 600 is displayed to be superimposed on the photographed image of the object E. In the region-of-interest designating unit 500, an instruction input for changing the size of the typical shape and designating the position of the typical shape is performed. In this embodiment, as the size change instruction for the typical shape, a size increase or reduction instruction is performed. The size of the typical shape of the three-dimensional region of interest can be changed stepwise. The typical shape corresponds to a predetermined three-dimensional shape in the present invention.

### (Display controlling unit)

The display controlling unit 600 outputs information for performing the display of the typical shape of the region of interest designated by the user. As an initial screen for the region of interest designation, the display controlling unit 600 displays the typical shape of the region of interest in a predetermined size. The display controlling unit 600 generates a display image of the region of interest according to a size change instruction and a position change instruction from the region-of-interest designating unit 500. In response to the size change instruction, the display controlling unit 600 generates image information in which the size of the typical shape of the region of interest is changed stepwise with a predetermined change amount. In response to the position change instruction, the display controlling unit 600 generates image information in which the display position is changed within a photographable range.

The display controlling unit 600 in this embodiment includes a region calculating unit that converts a display coordinate system and a scanning coordinate system. The display controlling unit 600 once changes, on the basis of the scanning coordinate system, the position and the size of the region of interest that is changed according to a position change and a size change of the three-dimensional region of interest designated by the region-of-interest designating unit. The display controlling unit 600 outputs, using the region calculating unit, image data of the display coordinate system such that screen display suitable for the display unit 601 is performed.

A scan track in this embodiment is a spiral track on an XY plane. That is, in this embodiment, photoacoustic measurement is performed such that a moving distance is an equal interval while spirally moving the supporting unit. Note that the spiral movement indicates that the entire supporting unit spirally moves and does not indicate that a rotational motion of the supporting unit with the center fixed like a top. Consequently, measurement is performed such that the high-sensitivity region is located at an equal interval in the measurement region. The influence of sensitivity unevenness in the measurement can be reduced. For the same purpose, an interval of circumferences (a circumferential pitch) of the spiral is also an equal interval. In this embodiment, the circumferential pitch of the spiral is set to a 10 mm pitch. In this case, the shape of an overall region of a high-resolution region formed by relatively spirally scanning the high-resolution region with respect to the object can be approximated to a column. Therefore, the typical shape of the region of interest in this embodiment is a columnar shape. Note that the circumferential pitches of the spiral scanning may be different rather than the equal interval.

In this embodiment, the typical shape is the columnar shape to simplify explanation. However, the typical shape may be a shape accurately representing an overall shape of the high-resolution region formed by the scanning or may be approximated to another shape. For example, the overall shape of the high-resolution region can also take a complicated shape obtained by rounding a flexible cylinder in a spiral shape. If the circumferential pitch of the spiral track increases, the overall shape of the high-resolution region is more complicated. These complicated shapes may be approximated by a column as long as there is no large difference in outlines or may be approximated as a flat barrel shape as another shape example.

### (Scanning region setting unit)

The scanning region setting unit 700 is a device that sets scanning regions in X, Y, and Z directions in which the supporting unit 400 is moved. The scanning region setting unit 700 sets X, Y, and Z regions in which the supporting unit 400 is moved such that the three-dimensional region of interest set by the region-of-interest designating unit 500 is included inside a track range of the high-resolution region G formed by scanning the supporting unit 400 in the X, Y, and Z directions. In this embodiment, a system for calculating a Z coordinate position in which the region of interest fits and spirally scanning the XY plane is adopted.

Note that, when scheduling scanning of the detector on the basis of the set region of interest, the scanning region setting unit 700 preferably sets, as the scanning region, a region slightly wider than the region of interest. This is because, to satisfy desired image quality concerning the set region of interest, it is preferable to acquire an acoustic wave from the periphery of the region of interest. However, compared with a measurement time assumed from the set region of interest, an actual measurement time of the scanning region more often increases.

### (Scanner)

The scanner 800 is a device that moves the position of the supporting unit 400 in the X, Y, and Z directions in FIG. 1 to thereby change the position of the supporting unit 400 with respect to the object E. Therefore, the scanner 800 includes guide mechanisms in the X, Y, and Z directions, driving mechanisms in the X, Y, and Z directions, and a position sensor that detects the positions in the X, Y, and Z directions of the supporting unit 400, all of which are not shown in the figure. As shown in FIG. 1, since the supporting unit 400 is mounted on the scanner 800, it is preferable to use, as the guide mechanisms, linear guides or the like resistible against a large load. As the driving mechanisms, lead screw mechanisms, link mechanisms, gear mechanisms, hydraulic mechanisms, and the like can be used. For a driving force, a motor or the like can be used. As the position sensor, an encoder, a potentiometer including a variable resistor, and the like can be used. The scanning unit of the present invention corresponds to the scanning region setting unit and the scanner.

### (Signal processing unit)

The signal processing unit 900 has a function of storing electric signals input from the acoustic detecting elements 300. The signal processing unit 900 has a function of generating characteristic information such as an optical characteristic in the object E using the electric signals input from the acoustic detecting elements 300 and generating an image of the inside of the object E on the basis of the characteristic information. Further, the signal processing unit 900 has a function of operating the photoacoustic apparatus such as light emission control of the light source 100 and driving control of the scanner 800.

An arithmetic unit of the signal processing unit 900 is typically configured from an element such as a CPU, a GPU, or an A/D converter and a circuit such as an FPGA or an ASIC. Note that the arithmetic unit may be not only configured from one element and one circuit but also configured from a plurality of elements and a plurality of circuits. Any one of the elements and any one of the circuits may execute kinds of processing performed by the signal processing unit 900.

A storing unit in the signal processing unit 900 is typically configured from a storage medium such as a ROM, a RAM, or a hard disk. Note that the storing unit may be not only configured from one storage medium but also configured from a plurality of storage media.

The signal processing unit 900 is preferably configured to be capable of simultaneously pipeline-processing a plurality of signals. Consequently, it is possible to reduce time until object information is acquired.

Note that the respective kinds of processing performed by the signal processing unit 900 can be stored in the storing unit as computer programs to be executed by the arithmetic unit. However, the storing unit in which the programs are stored is a non-transitory recording medium. Note that the display controlling unit, the scanning region setting unit, and the signal processing unit may be respectively configured of separate circuits and information processing device or may be configured as functional blocks of one information processing device.

### (Acoustic matching material)

The acoustic matching material 1000 is a material for filling a space between the object E and the acoustic detecting elements 300 and acoustically combining the object E and the acoustic detecting elements 300. The material is desirably fluid that has acoustic impedance close to the acoustic impedances of the object E and the acoustic detecting elements 300 and transmits the pulsed light generated by the light source 100. Specifically, water, castor oil, gel, or the like can be used.

### (Typical shape of the three-dimensional region of interest)

In this embodiment, the scanning of the supporting unit 400 is a spiral track. A region formed together with the track of the high-resolution region G at this point is a photoacoustic data acquisition region. In this embodiment, the data acquisition region formed by the track of the high-resolution region G is the typical shape of the three-dimensional region of interest. That is, photoacoustic data of a desired region can be acquired by instructing a size and a position change such that a range desired to be measured is fit on the inner side of the typical shape.

In this embodiment, to simplify explanation, a region approximated to a cylindrical shape is the typical shape of the three-dimensional region of interest. However, in implementation, a region smaller than an actual measurement region may be represented by an approximate shape or a shape itself of an aggregate of actual tracks of high-sensitivity regions G may be reproduced.

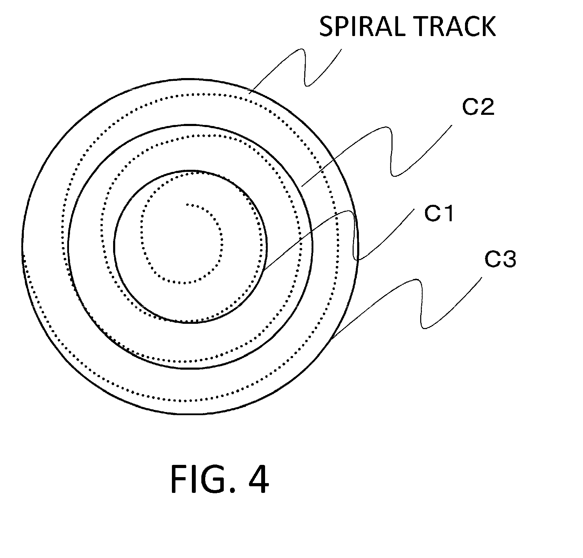

FIG. 4 is a diagram showing a state in which the data acquisition region changes by a predetermined amount every time a spiral increases by one circumference. The predetermined amount is a value that can also be called feature value corresponding to a pitch of a spiral track. This value is a feature value peculiar to the apparatus determined with reference to the configuration of the scanning unit of the supporting unit.

C1 in FIG. 4 is an XY section of the typical shape based on a spiral track of an innermost circumference. Since the circumferential pitch of the spiral track is the 10 mm interval as explained above, as the size of the XY section of the typical shape, a diameter increases by 10 mm like C2 and C3 every time the spiral is increased by one circumference. In this way, with a change amount corresponding to the number of circumferences of the spiral track set as a predetermined unit (in this embodiment, an integer times of a 10 mm pitch), the size change of the three-dimensional region of interest is instructed. When the user instructs an increase or reduction of the size of the three-dimensional region of interest, a region of interest at the time when the spiral track is increased or reduced by one circumference is calculated.

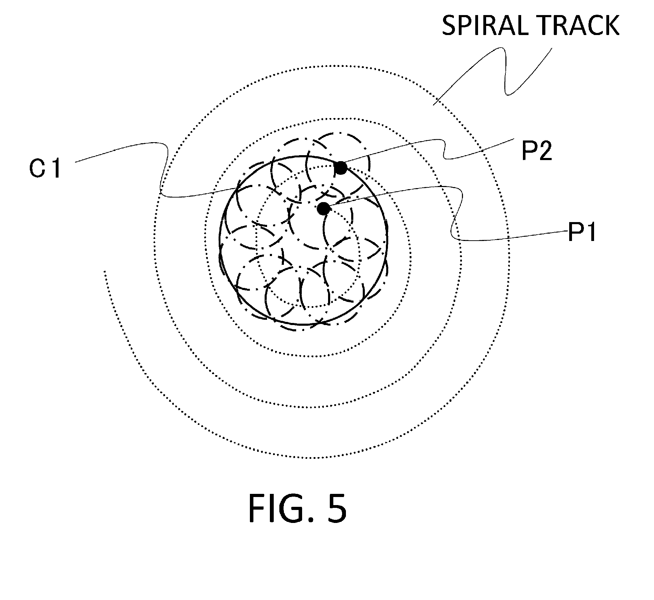

FIG. 5 is a diagram showing a sectional shape C1 on the XY plane of the region of interest in the spiral track. A circle C1 is a circle inscribing an aggregate of tracks of the high-sensitivity regions G formed up to an outer circumference immediately outside the spiral track. In the figure, a dotted line indicates the spiral track. Alternate long and short dash lines indicate the high-sensitivity regions G in respective measurement positions formed when measurement is performed at an equal interval pitch on the spiral track. P1 indicates a first sampling point of the innermost circumference. A sampling point corresponding to a first circumference from P1 is P2. At this point, the circle C1 is set to inscribe an aggregate of the high-sensitivity regions G formed in one circumference of the spiral track. Every time the spiral track increases by one circumference, not-shown circles C2 and C3 are set in the same manner. Then, concentric circles expanding at a 10 mm pitch are obtained.

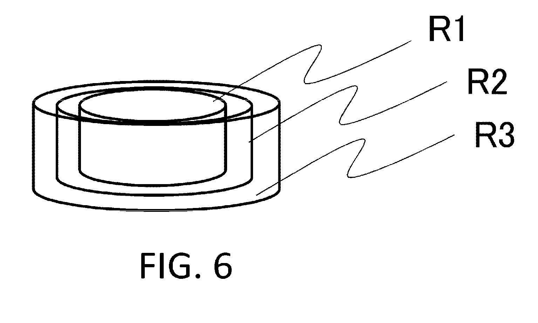

In this embodiment, as explained above, the typical shape is changed according to designation of expansion and reduction of the region of interest. A change in the region of interest is three-dimensionally shown in FIG. 6. R1 is a cylindrical region formed by the region of interest C1 on the innermost circumference side. R2 and R3 are cylindrical regions based on C2 and C3. When expansion by one stage is instructed for the region of interest of R1, the R2 region extended by one circumference of the spiral track is displayed. When expansion of one more stage is instructed, the R3 region further extended by one circumference of the spiral track is displayed.

### (Setting and measurement of the three-dimensional region of interest)

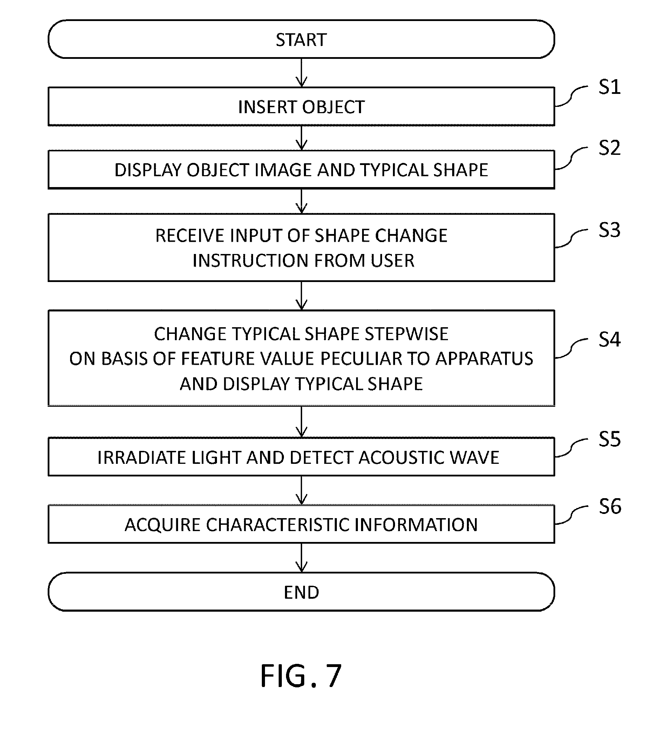

A process for deciding the three-dimensional region of interest and an acquisition method for a photoacoustic wave carried out on the basis of the set region of interest are explained. Steps of a flowchart of FIG. 7 are referred to according to necessity.

First, the object E is inserted into a container of the supporting unit 400 and the acoustic matching material 1000 is filled (step S1).

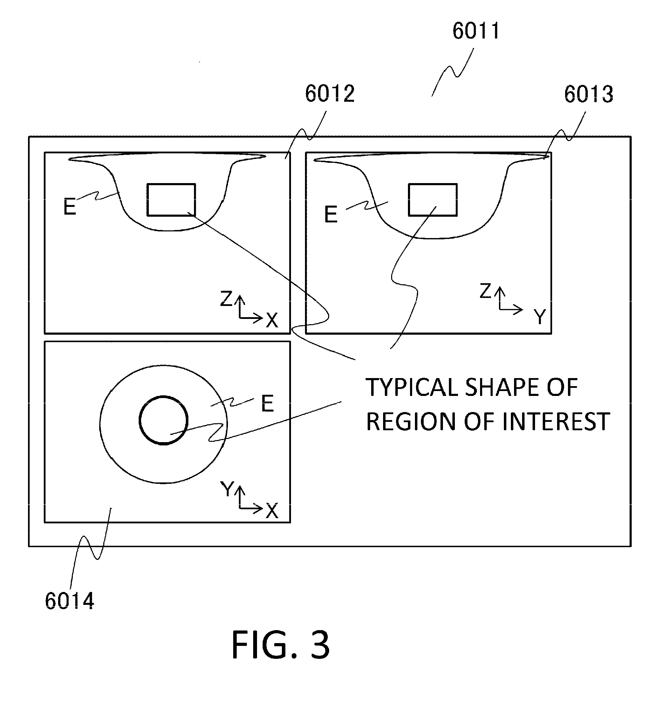

An example of an initial screen of the display unit 601 in this embodiment is shown in FIG. 3. On the display unit 601, as an initial screen 6011, capture camera images (reference numerals 6012, 6013, and 6014) of the object E are displayed. On the camera images, a typical shape of a three-dimensional region of interest is superimposed and displayed in an initial size (step S2).

A display screen in this embodiment is observation images from three directions by not-shown three capture cameras. However, the display screen may be images observed from two directions or may be a superimposed image of the typical shape of the three-dimensional region of interest with respect to a perspective view of the apparatus by simplified illustration.

The size of the typical shape (the three-dimensional region of interest) can be changed according to an expansion or reduction instruction of the user. Specifically, the user inputs an increase or reduction instruction for the size of the typical shape of the three-dimensional region of interest while viewing the capture camera images of the object E (step S3). The display controlling unit 600, which receives the size increase or reduction instruction for the region of interest, changes the size of the three-dimensional region of interest with a predetermined change amount based on a change amount of the measurement region in which the spiral track is increased or reduced in a unit of one circumference. The display controlling unit 600 updates the display of the display unit 601 with an image obtained by superimposing a three-dimensional region of interest obtained anew on the capture camera images of the object E (step S4).

The change of the typical shape at this point is performed stepwise with reference to a feature value (e.g., a pitch of the scanning unit) peculiar to the apparatus. That is, as shown in FIG. 6, the size change of the three-dimensional region of interest is performed by expanding or reducing the cylindrical region of interest with reference to the pitch 10 mm for one circumference of the spiral track and updating the region of interest. For the purpose of causing the user to be aware that the size of the typical shape is changed stepwise, a shape of a user interface displayed on the screen 6011 may be determined. For example, if a button with an upward arrow displayed thereon and a button with a downward arrow displayed thereon are displayed on the screen, the user can easily select an increase or reduction of the size. Alternatively, it is also preferable to select an increase or reduction with a wheel of a mouse.

At the same time, the user can select the three-dimensional region of interest displayed on the display unit 601 with input means, perform an input for moving a position relatively to the object E, and adjusts the position of the region of interest. The position adjustment may be performed before the size of the region of interest is decided. At this point, the input of the position moving instruction may be selection and drag operation by a touch panel of a pointing device such as a mouse or may be designation by an actual coordinate input or cursor operation.

When the user ends the selection of the three-dimensional region of interest, photoacoustic measurement for the set three-dimensional region of interest is started (step S5).

The scanning region setting unit 700 calculates a spiral track range of the supporting unit 400 on the basis of the set three-dimensional region of interest. The scanning region setting unit 700 receives, concerning the three-dimensional region of interest designated by the region-of-interest designating unit, an input of a coordinate value of the region of interest and size information indicating how many stages of expansion or reduction is performed with respect to a default size of the region of interest, calculates a measurement range, and decides a scanning track. When an instruction for moving the region of interest is given, the scanning region setting unit 700 also reflects the instruction on the scanning track.

The scanner 800 moves the supporting unit 400 according to the scanning track decided by the above procedure. Photoacoustic measurement is executed as explained below in positions during the movement. Note that light emission and acoustic wave detection may be carried out in stop positions while the supporting unit repeats a stop and movement or may be carried out while the supporting unit continuously moves.

In a first measurement position, light irradiated by the light source 100 using the optical system 200 is made incident on the object E via the acoustic matching material 1000. As a result, a photoacoustic wave is generated by a photoacoustic effect due to the light absorbed in the object E.

The photoacoustic wave generated in the object E is received by the acoustic detecting elements 300 via the acoustic matching material 1000 and converted into an electric signal.

The converted electric signal is sent to the signal processing unit 900 and stored in a storage device such as a memory in association with measurement position information. Consequently, a first electric signal in the first measurement position is stored.

Subsequently, the supporting unit 400 is moved by the scanner 800 to a second measurement position present on the inner side of the scanning region set by the scanning region setting unit 700. In the same manner as the measurement in the first measurement position, a second electric signal in the second measurement position is acquired and stored.

Thereafter, according to a process same as the process explained above, electric signals in all measurement positions in the scanning region set by the scanning region setting unit 700 are acquired. Images of measured regions are generated by the signal processing unit 900.

As explained above, the apparatus in this embodiment presents the typical shape of the three-dimensional region of interest to the user. The user viewing the typical shape of the three-dimensional region of interest can set the three-dimensional region of interest by increasing or reducing the size of the predetermined shape with the predetermined change amount. As a result, it is possible to easily set the three-dimensional region of interest. Consequently, in the setting of the region of interest, the user can set the three-dimensional region of interest while being aware of the scanning region without directly being aware of the change amount of the measurement range related to the design and the configuration of the apparatus. As a result, it is possible to reduce a problem in that a measurement time is long because a set region is slightly large and in that an unexpected unnecessary region is scanned.

### [Modification]

In this modification, a method of extending the configuration explained in the first embodiment to another form while maintaining the essence of the present invention is explained. In this modification, in particular, a scanning method of the supporting unit is different from the scanning method in the first embodiment.

In acquiring an acoustic wave generated from the object, the method of moving the supporting unit is not limited to the spiral shape. For example, a wide region can be measured even if the method is a scan method for repeating main scanning for moving the supporting unit in a long distance from an end to an end of the region of interest and sub-scanning for moving the supporting unit in a direction crossing (typically, orthogonal to) the main scanning direction. In this case, measurement concerning a stripe, which is an elongated beltlike region, is performed every time the main scanning is performed once and a plurality of the stripes are connected in the sub-scanning direction to measure the entire region of interest. In the case of this modification, the stripes are laid one on top of another in order to cover the region of interest. Therefore, the feature value peculiar to the apparatus is a value based on the shape and the size of the stripes.

In such vertical and horizontal scan, when the user instructs a size change for the shape of a typical three-dimensional region of interest displayed to be superimposed on an initial object image, a situation same as the situation in the first embodiment occurs. That is, when the user instructs size expansion, if an expanded portion protrudes from a stripe of the initial three-dimensional region of interest, an increase in the number of stripes to be scanned is necessary.

Therefore, in this modification as well, a method of easily setting a region while being aware of a scanning region can be presented to the user by changing the size of the region of interest stepwise according to an input value of the user from the input unit and a change amount peculiar to the apparatus. As a result, convenience for the user is improved. It is possible to reduce a measurement time and reduce a burden on an examinee.

### Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

Provided is an object information acquiring apparatus including: detecting elements converting an acoustic wave propagated from an object to an electric signal, a processing unit acquiring characteristic information of the object using the electric signal, a supporting unit supporting the detecting elements, an input unit receiving information concerning three-dimensional region of interest, a display controlling unit displaying a predetermined three-dimensional shape representing, a scanning region setting unit setting a
scanning region, and a scanning unit moving the supporting unit, wherein the display controlling unit changes the size of the region of interest stepwise by using a feature value peculiar to the apparatus as a unit based on the inputted information, and displays the changed region of interest.

## Claims

1. An object information acquiring apparatus comprising:
a light source;
an irradiating unit configured to irradiate an object with light from the light source;
a plurality of detecting elements configured to detect an acoustic wave propagated from the object and output an electric signal;
a processing unit configured to acquire characteristic information of an inside of the object using the electric signal;
a supporting unit configured to support the plurality of detecting elements;
an input unit capable of receiving an input of information concerning a region of interest, which is a three-dimensional region where the characteristic information is to be acquired;
a display controlling unit configured to cause a display unit to display a predetermined three-dimensional shape representing the region of interest;
a scanning region setting unit configured to set a scanning region on the basis of the region of interest; and
a scanning unit configured to move the supporting unit in the scanning region, wherein
the display controlling unit changes, on the basis of the information concerning the region of interest input from the input unit, a size of the region of interest stepwise by using a feature value peculiar to the apparatus as a unit, and causes the display unit to display the region of interest after the change.

2. The object information acquiring apparatus according to claim 1, wherein
the input unit receives an instruction for setting the scanning region on the basis of the region of interest displayed on the display unit, and
when receiving the instruction for setting the scanning region input from the input unit, the scanning region setting unit sets the scanning region on the basis of the region of interest displayed on the display unit.

3. The object information acquiring apparatus according to claim 1 or 2, wherein the display controlling unit causes the display unit to display the scanning region set by the scanning region setting unit.

4. The object information acquiring apparatus according to any one of claims 1 to 3, wherein the scanning unit spirally moves the supporting unit.

5. The object information acquiring apparatus according to claim 4, wherein
the predetermined three-dimensional shape representing the region of interest is a columnar shape, and
the feature value peculiar to the apparatus is a circumferential pitch of the spiral.

6. The object information acquiring apparatus according to any one of claims 1 to 5, wherein the input unit can receive designation of an increase or reduction of a size of the predetermined three-dimensional shape.

7. The object information acquiring apparatus according to any one of claims 1 to 6, wherein the input unit can receive designation of movement of the predetermined three-dimensional shape.

8. The object information acquiring apparatus according to any one of claims 1 to 7, wherein the supporting unit supports the plurality of detecting elements such that directional axes of at least a part of the plurality of detecting elements converge.

9. The object information acquiring apparatus according to claim 8, wherein the scanning region setting unit sets the scanning region such that the region of interest includes high-sensitivity regions where the directional axes of the plurality of detection elements converge.

10. The object information acquiring apparatus according to claim 8 or 9, wherein
the scanning unit spirally moves the supporting unit, and
the predetermined three-dimensional shape representing the region of interest is a shape of an aggregate of tracks of the high-sensitivity regions.

11. The object information acquiring apparatus according to any one of claims 1 to 10, further comprising the display unit.
